Europäisches Patentamt

European Patent Office

Office européen des brevets

(11) Publication number: **0 048 283**

**B1**

# (12) EUROPEAN PATENT SPECIFICATION

(45) Date of publication of patent specification: **16.05.84**

(21) Application number: **81900752.7**

(22) Date of filing: **25.03.81**

(86) International application number:
**PCT/JP81/00062**

(87) International publication number:
**WO 81/02674 01.10.81 Gazette 81/23**

(51) Int. Cl.³: **A 61 K 35/23,** A 61 K 35/50,
A 61 K 37/00, A 61 K 45/02,
C 07 G 7/00

(54) **VIRUS-INHIBITING SUBSTANCE AND PROCESS FOR PREPARING THE SAME.**

(30) Priority: **26.03.80 JP 37494/80**

(43) Date of publication of application:
**31.03.82 Bulletin 82/13**

(45) Publication of the grant of the patent:
**16.05.84 Bulletin 84/20**

(84) Designated Contracting States:
**FR**

(56) References cited:
**EP-A-0 000 520**
**JP-A-52 038 009**
**JP-A-52 051 009**

(73) Proprietor: **TAGUCHI, Fumiaki**
**14-1, Yokodai 5-chome Sagamihara-shi
Kanagawa 229 (JP)**

(72) Inventor: **TAGUCHI, Fumiaki**
**14-1, Yokodai 5-chome Sagamihara-shi
Kanagawa 229 (JP)**

(74) Representative: **Jolly, Jean-Pierre et al
Cabinet BROT 83, rue d'Amsterdam
F-75008 Paris (FR)**

Courier Press, Leamington Spa, England.

## Description

### Field of the art

This invention relates to a novel interferon and also to a process for producing the same. More particularly, the present invention pertains to a human epithelial cells interferon (hereinafter sometimes referred to as HE—IF) exhibiting an especially potent antiviral activity in human epithelial cells, which is produced in human epithelial cells such as ones from amnion, kidney or amniotic fluid, and also to a process for producing the same.

The interferon according to the present invention is considered to belong to Type I interferon in a broad sense, but it is a novel interferon different in reactivity with antibodies against human leukocyte interferon and human fibroblast interferon.

### Background of the art

Generally speaking, interferons may be classified broadly into Type I and Type II. As the Type I interferons, there have been known fibroblast interferon (hereinafter referred to as HF—IF) produced by fibroblasts and leukocyte interferon (hereinafter referred to as HL—IF) produced by blood cells such as leukocyte, lymphocyte and lymphoblast. The Type II interferon is instable and can be produced only in a very low yield, and for this reason, the properties of Type II interferon have not yet been clarified sufficiently. In contrast, the Type I interferon as mentioned above has attracted attention as useful for therapeutical purposes. Thus it is reported that HL—IF can effectively act on lymphoblasts of Burkitt's lymphoma and also that HF—IF can effectively act on cells of osterosarcoma.

However, there have been neither reports on nor suggestions for predicting the presence of an interferon which can exhibit excellent activity on epithelial cells covering all over the body surface, among cell populations, including blood cells, skeletal cells (muscle cells), substantive cells of respective organs and epithelial cells.

Although it is generally known from EP—A—520 that interferon can be produced by most cells, for example white blood cells, fibroblasts lymphoblastoid cells, cells of epithelial types such as kidney cells or Hela cells, myeloma cells, no distinction is made between such cells as regards the type of interferon that can be obtained.

### Disclosure of the invention

The present inventors have made investigations to discover an epithelial cells interferon exhibiting an antiviral activity specifically on epithelial cells. Consequently, it has now been found that there can be produced a novel interferon from amnion epithelial cells, kidney epithelial cells and amniotic fluid epithelial cells, which exhibits a potent activity for inhibiting multiplication of virus specifically in epithelial cells and yet has antigenic properties different from those of HL—IF and HF—IF, and which therefore should be designated under a new name. Purification as well as characterization of the properties of this interferon were also conducted to confirm its novelty.

Thus, the novel interferon according to the present invention is produced in human epithelial cells and is distinguished from human fibroblast interferon in reactivity with anti-HL—IF antibodies or anti-HF—IF antibodies or both.

The specific feature of the process for producing the novel interferon according to the present invention comprises bringing human epithelial cells into contact with an interferon inducer and then cultivating said epithelial cells in a medium in the presence or the absence of serum thereby to produce an interferon exhibiting very potent antiviral activity in human epithelial cells.

The interferon HE—IF according to the present invention is very stable, high in viral growth inhibition and also has a specific activity different from conventional Type I interferons with respect to the strong antiviral activity exhibited specifically in human epithelial cells. In other words, the interferon HE—IF exhibits an antiviral activity in epithelial cells which is greater by far than in fibroblasts. Further, the activity can be exhibited more rapidly and more strongly in epithelial cells than in fibroblasts, indicating that the present interferon has characteristics not found in those known in the art. Because of these features, this interferon can be expected to exhibit excellent pharmacological effects especially with respect to diseases such as viral infections, malignant tumors, etc., whose sources can be found in epithelial cells.

It is also another advantage of the present invention that the human epithelium, to be used as starting material for production of interferon according to the present invention, are available with relative ease.

### Brief description of the drawings

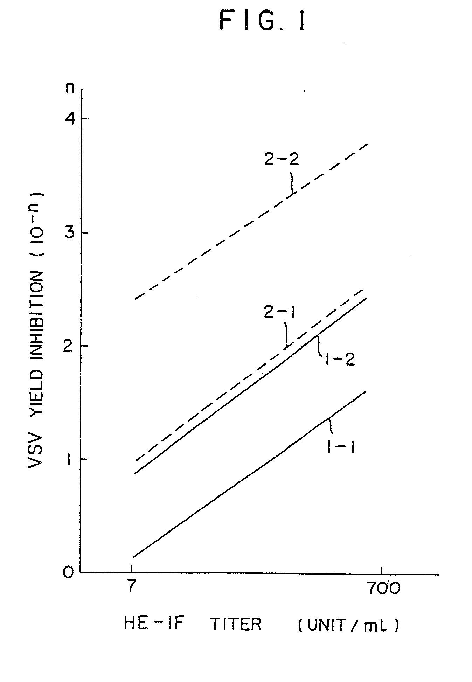

Fig. 1 shows the yield inhibition by HE—IF of Vesicular stomatitis virus (abbreviated as VSV), the titers of HE—IF being plotted on the abscissa against the yield inhibition on ordinate, in which:

1—1 is the case when HEL—R66 cells are treated with HE—IF for 5 minutes and then infected with the virus;

1—2 the case when HEL—R66 cells are treated with HE—IF for 360 minutes and then infected with the virus;

2

2—1 the case when PHA cells are treated with HE—IF for 5 minutes and then infected with the virus; and

2—2 the case when PHA cells are treated with HE—IF for 360 minutes and infected with the virus; and

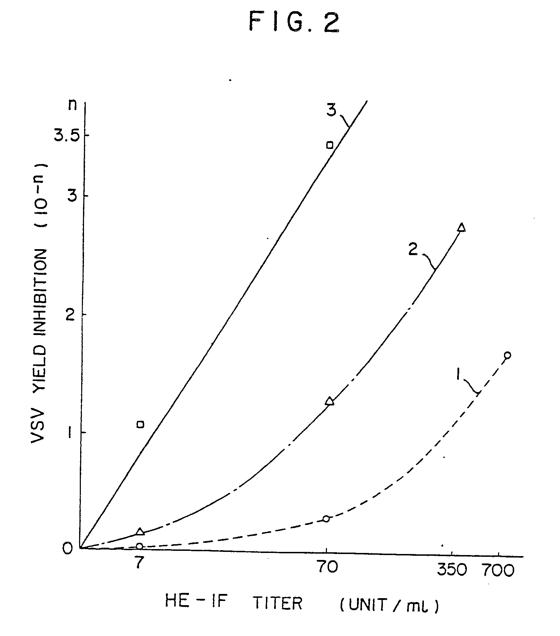

Fig. 2 shows the VSV yield inhibition when the inoculation of VSV and HE—IF is performed simultaneously, in which:

1 is the case when HEL—R66 is used;

2 the case when HEL is used; and

3 the case when PHA is used.

The best mode for practice of the invention

1. *Production of the interferon*

The interferon according to the present invention is produced by first contacting human epithelial cells with an interferon inducer and then cultivating said epithelial cells in a medium in the presence or the absence of serum.

1) Human epithelial cell source

As human epithelial cells, various kinds of epithelial cells can be used. Typical examples are those derived from amnion, amniotic fluids and kidney. Among them, amnion is preferred in view of its availability and ease of maintenance and also because of its being a mass of cells capable of producing IF with a higher titer.

2) Interferon inducer

As a substance which acts on living cells to induce interferons therein, namely, an interferon inducer, viruses, bacteria, components of fungi and bacteria, nucleic acids, polysaccharides, etc., have been known. In the present invention, it is possible to use any desired species capable of exhibiting the effect of the present invention (the inducing effect differing, depending on the respective species).

A typical interferon inducer to be used in the present invention is a virus. As such a virus, it is possible to use any of paramyxoviruses and orthomyxoviruses. The viruses suitably used in the present invention may include Newcastle disease virus (hereinafter abbreviated as NDV), Sendai virus, Influenza virus, etc.

A virus which has been inactivated by ultraviolet rays or heat may also be employed.

3) Induction of interferon

Human epithelial cells may be contacted with an interferon inducer as mentioned above according to any method which can ensure inducing of interferon.

Specific contacting methods would be different depending on the inducer employed (and the epithelial cells employed). When the inducer is a virus, it is a common practice to shake a dispersion of the virus with epithelial cells, for example, amnion suitably cut into pieces to adsorb the virus thereon. The epithelial cells may either consist of single cells or be in the form of a membrane suspended in a medium to be used in the succeeding step. The contact temperature and time may be at any desired levels sufficient to effect inducing of interferon, and specific values thereof can be readily selected by those skilled in the art on the basis of the examples set forth hereinafter.

4) Culture medium and incubation conditions

As a base medium to be used for maintenance of human epithelial cells and production of HE—IF, use may be made of a commercially available complete synthetic medium containing saccharides, amino acids, vitamins and inorganic salts, as exemplified by Eagle's minimum essential medium (MEM), RPMI (Roswell Park Memorial Institute) 1640 medium, M199 medium etc. (E. H. Lennette & N. J. Schmidt "Diagnostic Procedures for Viral and Rickettsial Infections (4th ed.)", 1969, Am. Public Health Assoc. N.Y., U.S.A).

Generally speaking, addition of a serum is deemed to be indispensable for production of interferon, but it is also possible in the present invention to use a medium to which no serum is added.

The cultivation conditions can be selected appropriately for the production of the interferon according to the present invention. The cultivation temperature is generally 37°C, at which cultivation is conducted for 38 to 58 hours, and thereafter the cultivation temperature is lowered to $27 \pm 2°C$, whereby the yield of interferon can be increased to a great extent. The cultivation period may be in any case suitably within 2 to 4 days.

5) Accelerator for interferon production

In addition to the basic conditions for the production of HE—IF as stated above, the present inventors have investigated the effects of various additives. With the progress of the research, it was found that production of HE—IF in titer of 300% or more relative to that in the case of no addition can be realized by the addition of such additives on which there has been no report in the art, including pituitary hormones such as ACTH, xanthine derivatives such as caffeine, theophylline, theobromine, etc., alloxan, indole, indoleacetic acid, a barbiturate, amantadine, imidazole, carbachol, etc., and also insulin which has been reported as having a weak enhancement effect in the HF—IF production system.

The effect of these additives was found to be even more pronounced when they are added at suitably selected stages. For example, in case of insulin, it should preferably be added after the cultivation has continued for 12 to 36 hours when the temperature is maintained at 37°C over the entire cultivation procedure, or it should preferably be added at the beginning of cultivation when the temperature is maintained at 37°C for 48 hours, and thereafter lowered to 27°C.

These new findings concerning these accelerators and the aforesaid cultivation temperatures can be very effectively utilized for mass production of HE—IF. According to the method known in the art for production of interferon by a bulk production system, the amount of interferon at the maximum level is about 10,000 units/ml for HF—IF and about 30,000 units/ml for HL—IF, whereby interferon produced by one cell is about 0.15 unit or less. In contrast, according to the process of the present invention, it is possible to produce HE—IF in an amount of about 2,000,000 units/ml, whereby interferon produced per cell is as much as 10 units.

6) Purification of the interferon obtained

Purification of HE—IF produced according to the process of the present invention may be performed according to the methods well known in the art which are utilized for purification of known interferons, including human interferons such as HF—IF, HL—IF, or mouse interferons. For example, it is possible to obtain a purified product of $10^6$ units/mg of more by column chromatography in which SP Sephadex (registered Trade Mark) or Blue Sepharose (registered Trade Mark), are used.

HE—IF is found to be relatively stable under physical and chemical treatments. In particular, when no serum is used for the production of HE—IF, the resultant interferon is free from serum mixed therein, which is advantageous in purification as compared with conventional interferons.

2. *Properties and activity of the interferon according to the present invention*
(1) Stability (comparison with HF—IF and HL—IF)

Table 1 shows the stability of HE—IF in comparison with those of HF—IF and HL—IF. It can be seen that HE—IF is more stable than HF—IF.

TABLE 1

| Treatment conditions | HE – IF | HF – IF | HL – IF |
|---|---|---|---|
| pH 2.0 – 10.0, 4°C two days | Stable | Slightly unstable | Stable |
| pH 7.0, 4°C, two days | Stable | Stable | Stable |
| pH 7.0, 37°C, 24 hrs. | Stable | Unstable | Stable |
| pH 7.0, 56°C, 30 min. | Relatively stable (Stable in presence of SDS) | Unstable | Stable |
| Storage under freezing for one week: | | | |
| −40°C | Stable | Stable | Stable |
| −70°C | Stable | Stable | Stable |
| Freeze-thaw treatment (−70°C, 3 times) | Relatively stable | Unstable | Relatively stable |
| Treatment with trypsin (100 μg/ml) | Unstable | Unstable | Unstable |

(2) pH stability

Sample solutions, each comprising 3 ml of amnion interferon of an activity of 14,000 units/ml containing or not containing 0.1% of sodium dodecyl sulfate (hereinafter referred to as SDS), are adjusted to the treatment pH by 1 N HCl or 1 N NaOH, left to stand at 4°C for 48 hours, and adjusted again to pH 7. Then their residual activities were determined by using FL cells. The results are shown in the following table 2.

TABLE 2

| Treatment pH | Residual activity (units/ml) | |
|---|---|---|
| | No SDS added | 0.1% SDS added |
| 2.0 | 2,400 (17.1) | 12,000 (85.7) |
| 3.0 | 3,000 (21.4) | 14,000 (100) |
| 4.0 | 3,000 (21.4) | 13,000 (92.9) |
| 7.0 | 6,800 (48.6) | 13,000 (92.9) |
| 9.5 | 4,600 (32.9) | 13,000 (92.9) |

In the brackets, the proportions of the residual activities after the treatment based on the activities before the treatment are shown in terms of %.

As is apparent from the above results, HE—IF is very stable at pH 2.0 to pH 9.5, its stability being further increased by the addition of 0.1% SDS.

(3) Temperature stability

The interferon activity of HE—IF was found to have disappeared almost completely as a result of the incubation at pH 7.0 at 56°C for one hour. In the presence of 0.04% sodium dodecyl sulfate, however, more than 80% activity was found to be remaining. Activity assay was carried out by using FL cells.

TABLE 3

| SDS concentration (%) | Residual activity (units/ml) |
|---|---|
| 0 | 10 |
| 0.008 | 500 |
| 0.04 | 1500 |
| 0.2 | 1100 |
| Control (no SDS added, 4°C) | 1800 |

5

(4) Species specificity

TABLE 4

| Species of cells | Induction of antiviral activity |
|---|---|
| Human cells (FL, HEL, FS, HEL—R66, PHA) | Yes |
| Monkey cells (Vero, LLCMK$_2$, BSC—1) | Yes |
| Dog cells (MDCK) | No |
| Rabbit cells (RK—13) | No |
| Mouse cells (L, L929, 3T3) | No |
| Hamster cells (BHK21) | No |
| Chicken embryo cells (CE) | No |

As can be seen from the above, HE—IF induces antiviral state in cells from human and monkey origins.

(5) Antiviral activity in various human cells

TABLE 5

| Cell line | Activity of HE–IF (units/ml) | |
|---|---|---|
| | Example 1 | Example 3 |
| HEL (fibroblast from embryonic lung) | 18* | 600* |
| HEL—R66 (cell-line from HEL) | 100 | 5,000 |
| FL (cell-line from amnion) | 400 | 20,000 |
| PHA (epithelial cells from amnion) | 3,500 | 500,000 |
| HEK (human embryo kidney cells) | 3,300 | 350,000 |

The values marked with asterisk were obtained by the CPE inhibition method, others by the plaque reduction method (activity is shown in terms of the IF dilution which inhibits 50% of VSV plaque formation in each cell as one unit/ml).

As shown above, FL cells belonging to epithelial cells exhibits 4 times, while PHA exhibits 35 to 100 times the activity assayed using HEL—R66 belonging to fibroblasts, thus indicating specifically potent activity in epithelial cells.

6

**0 048 283**

(6) The inhibition of VSV multiplication

The specific affinity of HE—IF for epithelial cells was also shown by a more rapid establishment of antiviral state in epithelial cells than in fibroblasts. That is, in each of the PHA cells as epithelial cells and HEL—R66 cells as fibroblast cells, 7 units/ml or 700 units/ml of HE—IF was added.

After 5 minutes or 360 minutes, the cells were washed to remove interferon and infected with VSV (moi = 0.1). These steps were then followed by incubation at 37°C. The supernatant medium was harvested, after 24 hours in the case of HEL—R66 cells or after 48 hours in the case of PHA cells, and the VSV yield in the supernatant was assayed by the use of HEL—R66 cells. The ratio of the infectious titer determined relative to the virus yield in the control without addition of HE—IF was calculated and taken to be the degree of inhibition of virus yield. The results are shown in Fig. 1, from which it can be seen that only five minutes' contact of PHA cells with HE—IF can exhibit the antiviral activity for inhibition of virus yield substantially equal to that resulting from the contact of HEL—R66 cells with HE—IF for 360 minutes.

Thus, since HE—IF has higher affinity for epithelial cells and can exhibit activity rapidly therefore, noticeable antiviral activity was also exhibited even when HE—IF was applied simultaneously with virus inoculation without previous contact with the cells. That is, each of the PHA cells, HEL cells and HEL—R66 cells was contacted with a mixture of VSV (moi = 0.1) and HE—IF (7, 70, 700 units/ml) for one hour. Then, the cells were washed to remove HE—IF and unadsorbed VSV, after which incubation was carried out at 37°C for 48 hours for PHA cells and for 24 hours for HEL cells and HEL—R66 cells. VSV yield in each supernatant was assayed by the use of HEL—R66, and the ratio relative to the virus yield in the control to which no HE—IF had been added was calculated and taken as the degree of inhibition of virus yields.

The results are as shown in Fig. 2, indicating that HE—IF can effeictively inhibit virus yield even when applied simultaneously with infection with virus in PHA cells.

In addition, also when HE—IF was applied after infection with virus without previous contact of HE—IF, a noticeable antiviral activity was observed.

That is, each of the PHA cells, HEL cells and HEL—R66 cells was inoculated with VSV (moi = 0.1), and the resultant cells were maintained at 37°C for 60 minutes thereby infecting them with the virus. Then, after washing the cells to remove unadsorbed VSV, the cells were divided into the following groups for treatment with HE—IF (5 units and 50 units), respectively.

1) Immediately treated with HE—IF for 60 minutes, then washed to remove HE—IF, and then incubated at 37°C for 24 hours.

2) Further maintained at 37°C for 60 minutes to permit progress of virus multiplication, then treated with HE—IF for 60 minutes, thereafter washed to remove the HE—IF, and finally cultivated at 37°C for 24 hours.

3) HE—IF was added immediately to the cells, and, without removal of the HE—IF, cultivation was conducted at 37°C for 24 hours. Each supernatant was collected for determination of VSV yield therein by the use of HEL—R66 cells, and the ratio was calculated relative to the virus yield in the control to which no HE—IF had been added and taken to be the degree of inhibition of virus multiplication. The results are shown in the following Table, indicating that HE—IF can effectively inhibit virus multiplication even when applied 60 to 120 minutes after inoculation of the virus in the PHA cells.

TABLE 6

| Application of HE—IF (after infection with VSV) | Units applied | VSV yield inhibition $(-Log_{10}PFU/ml)$ | | |
|---|---|---|---|---|
| | | PHA cells | HEL cells | HEL—R66 cells |
| 1) only 60 minutes immediately after infection | 5 | 1.3 | 0 | 0 |
| | 50 | 2.5 | 0.1 | 0 |
| 2) only 60 minutes after 60 minutes | 5 | 1.4 | 0 | 0 |
| | 50 | 2.7 | 0.1 | 0.1 |
| 3) 24 hours immediately after infection | 5 | 1.7 | 0 | 0 |
| | 50 | 4.4 | 1.1 | 0.7 |

7

This is an advantageous property when HE—IF is used as a medicine. There has been no report of an interferon exhibiting such an activity.

(7) Antigenic property

The specificity of interferon can be most positively indicated by the difference in antigenic property. Hence, the neutralization test as indicated in the following examples of experiments was conducted by using anti-HL—IF serum and anti-HF—IF serum. As the interferon samples as antigens, HL—IF prepared by use of human leukocytes, HF—IF prepared by use of human fibroblasts and HE—IF prepared by use of human amnion were employed. On the other hand, as the antiserum for respective interferon types (highly immune rabbit serum), use was made of immune serum against HL—IF (hereinafter referred to as anti-HL—IF serum) and immune serum against HF—IF (hereinafter referred to as anti-HF—IF serum). The antiviral activity of the interferon was assayed according to 50% reduction of VSV plaque formation in FL cells. For the neutralization tests, four sets of 4-fold serial dilutions of immune serum (2 ml), respectively, were prepared. On the other hand, antigens each adjusted to 10 units/ml were prepared for respective interferons. As to the details relating to the 50% reduction of plaque formation method, reference should be made to William E. Steward II: The Interferon System, 1979, Springer-Verlag, Wien-N.Y. and R. R. Wagner: Virology *13*, 323 (1961). In the dilutions for each immune serum, HL—IF was added to the first series, HF—IF to the second, HE—IF to the third and the diluent (MEM medium containing 0.5% serum) was mixed in equal amounts with immune serum, respectively. Thereafter, the reaction of each mixture incubated at 37°C was carried out in a water bath for 60 minutes. The resultant mixture was diluted with the diluent to 5, 15 and 45 times, and all of the dilutions were added, each in a quantity of 1 ml, to FL cells in a plastic plate (12 wells). Then cultivation was carried out at 37°C in a $CO_2$ incubator overnight. Then, after the culture was washed well, it was inoculated with VSV (50 plaques/well), and cultivation was further carried out at 37°C for 2 days to form plaques. After addition of Neutral Red, the number of plaques was counted, and the activity of serum (dilution times) capable of neutralizing the antiviral activity of 10-unit interferon was calculated therefrom. The antigenic property was consequently approximated from the final dilution of immune sera for respective types. The results are given in Table 7.

TABLE 7

|  | Neutralization antibody titer of immune sera for respective types | |
|---|---|---|
|  | Anti—HL—IF serum | Anti—HF—IF serum |
| HL—IF | 30.000 | <250 |
| HF—IF | <250 | 20,000 |
| HE—IF | <250 | 1,000 |

As seen from these results, HE—IF has a reactivity with antibodies different from any of those of known interferons HL—IF and HF—IF.

(8) Physicochemical properties

The molecular weight of HE—IF prepared according to the process of the present invention was determined by the gel filtration method to be about 25,000 which is substantially identical with that of chymotrypsinogen. When the isoelectric point was measured by the isoelectric point electrophoresis method, the activity was observed at around pH 6.

(9) Adsorption characteristic

The HF—IF is strongly adsorptive on glass ware and when a solution containing HF—IF is transferred to new glass vessels repeatedly, most of activity is known to be lost through adsorption on glasses. In contrast, as shown in Table 8 below, no such phenomenon is observed in the case of HE—IF.

8

TABLE 8

| Number of transfer | Residual activity of HE—IF (unit/ml) |
|:---:|:---:|
| 0 | 1500 |
| 1 | 1500 |
| 2 | 1500 |
| 4 | 1500 |
| 6 | 600 |

3. *Quantitative titration of the interferon of the present invention*

In order to confirm the potent viral inhibiting effect of the novel interferon HE—IF according to the present invention, the present inventors have established a supersensitive method for quantitative determination of HE—IF by the use of primary amnion cells.

In the prior art, for quantitative determination of human interferons, FL cells (cell-line from amnion) or FS cells (fibroblasts from human foreskin) have been used without specific consideration for specificity of interferon on cells. In neither case was there employed a cell system reacting specifically with the interferon for more quantitative determination.

The present inventors have made extensive studies for a long time in order to discover a cell system suitable for quantitative determination of HE—IF activity. As a consequence, it has now been found that primary human amnion cells (hereinafter abbreviated as PHA cells), prepared by cultivation of the single cells obtained by the treatment of amnion with trypsin, are cell system exhibiting a surprisingly high sensitive reactivity with HE—IF. VSV was used as the virus. Thus, while there has been no report on the use of PHA cells for interferon assay, they can be utilized for a new method for quantitative determination of interferon activity according to the process of the present invention. This quantitative determination method is described in detail below.

A fresh amnion is cut into pieces of suitable sizes, which are then treated in a conventional procedures with 0.25% trypsin solution and thus dispersed into single cells. The cells are collected by centrifugation at 1,000 rpm for 10 minutes and further adjusted to a concentration of $10^6$ cells/ml by using a culture medium of M 199 to which 20% calf serum has been added. Then, 1 ml of the cell suspension is apportioned into each well in a plastic plate (e.g. "Multi-well plate", 12 wells, flat-bottomed; Linbro Sci. Co.). The plate is placed in a carbon dioxide incubator for cultivation at 37°C for 4 to 5 days to form monolayers. The culture medium is thereafter removed by aspiration, and the residue is amply washed with Hanks' solution. Subsequently, an interferon sample diluted with M 199 medium containing 2% calf serum is apportioned into each 1 ml/well, and the reaction is allowed to proceed at 37°C. After aspiratory removal of the interferon sample, each 0.5 ml/well is inoculated with VSV. VSV employed is adjusted by dilution with M 199 medium so as to form 50 plaques/well. After VSV is adsorbed at 37°C for 60 minutes, VSV is removed by washing and the M 199 medium (pH 7.2) containing 20% calf serum and 1.0% agar is apportioned into each 1 ml/well, after which cultivation is carried out at 37°C for 2 days. To each well, there is added Neutral Red solution (1:10,000). The number of plaques is counted and the interferon activity (units/ml) is determined in terms of the dilutions, which reduces plaques by 50% in comparison with the control.

When antiviral activity of HL—IF or HF—IF is assayed by this method, there is only an increase of antiviral activity of 1.8 to 2.3 times in the titers of IF determined by use of FL cells or HEL cells (fibroblasts for human embryonic lung). On the other hand, the values determined by use of PHA cells, of HE—IF are as high as 35 to 100 times as compared with the values determined by use of HEL cells or HEL—R66 cells (cell-line from human embryonic lung fibroblasts) and also as high as 8 to 20 times as compared with those determined by use of FL cells.

Thus, this method can be said to be a suitable method for quantitative determination of HE—IF activity.

The following examples are set forth for illustration of specific examples of practice of the present invention.

Example 1

A fresh human amnion was cut into pieces of about 10 cm² and contacted with NDV suspension in a proportion of 5 ml per 1 g of the piece of amnion. The NDV suspension employed contained 160 to 320 HA units/ml. By shaking at 37°C for 90 minutes, NDV was adsorbed onto the piece of amnion.

Then, only the piece of amnion was transferred into a culture medium of a quantity of 10 ml per 1 g of the piece of amnion. Incubation was then carried out at 37°C

As the culture medium, a commercially available M 199 medium was employed, and no serum was added thereto. In this Example 1, comparison was made between the tests in which various additives were added and a control in which no additive was added.

Some additives were added at the initiation of incubation, with sampling of culture medium after 24 or 48 hours, while others were added after 24 hours or 48 hours after the initiation of incubation, with sampling of culture medium after 48 hours or 72 hours.

The culture sample was adjusted to pH 2.0 with 1 N HCl, and stored under cooling at 4°C for 2 days to inactivate HDV. Then, it was adjusted to pH 7.0 with 1 N NaOH, and stored under freezing at −70°C. The frozen sample was thawed immediately before measurement to provide a crude HE—IF sample, which was then subjected to activity assay according to the method described above. The results are as shown in Table 9 below.

TABLE 9

| Additive | Concentration | Timing of addition | Interferon activity (unit/ml) | | |
|---|---|---|---|---|---|
| | | | 24 hours cultivation | 48 hours cultivation | 72 hours cultivation |
| None | | | $1.5 \times 10^5$ | $2.5 \times 10^5$ | $2.2 \times 10^5$ |
| Caffeine | 20 $\mu$g/ml | Initially | $7.5 \times 10^5$ | | |
| Xanthine | $10^{-1}$mM | Initially | $8.4 \times 10^4$ | $1.1 \times 10^6$ | $3.8 \times 10^5$ |
| Alloxan | $10^{-1}$mM | Initially | $3.8 \times 10^5$ | $8.4 \times 10^5$ | $9.3 \times 10^5$ |
| Indole-acetic acid | $10^{-2}$ mM | Initially | $9.3 \times 10^4$ | $9.2 \times 10^5$ | $6.5 \times 10^5$ |
| Indole | $10^{-2}$mM | Initially | $4.1 \times 10^5$ | $9.3 \times 10^5$ | $9.0 \times 10^5$ |
| | | 24 hrs | | $1.3 \times 10^6$ | $3.8 \times 10^5$ |
| Barbital | $10^{-1}$mM | Initially | $2.8 \times 10^5$ | $7.0 \times 10^5$ | $3.0 \times 10^5$ |
| Imidazole | $10^{-1}$mM | Initially | $2.2 \times 10^5$ | $3.8 \times 10^5$ | $7.1 \times 10^5$ |
| Carbacol | $10^{-1}$mM | Initially | $2.3 \times 10^5$ | $9.5 \times 10^5$ | $2.0 \times 10^5$ |
| Amantadine | $10^{-1}$mM | | $2.2 \times 10^5$ | $1.4 \times 10^6$ | $5.3 \times 10^5$ |
| ACTH | 1 international unit/ml | Initially | | $7.5 \times 10^5$ | |
| Insulin | 1 international unit/ml | 24 hrs. | | $1.5 \times 10^6$ | |

Example 2

When pieces of amnion treated similarly as in Example 1 with NDV had been incubated at 37°C for 48 hours and thereafter further incubated at 27°C for 72 hours, the titer of HE—IF was found to have increased. The results are shown in the Table 10 below. However, no noticeable effect was observed when incubation was conducted at 27°C for 72 hours after incubation at 37°C for 24 hours.

TABLE 10

| Incubation temperature | Incubation time (hr.) | Interferon activity (unit/ml) | | |
|---|---|---|---|---|
| | | 24 hrs. | 48 hrs. | 72 hrs. |
| 37°C | 0 – 72 | $1.6 \times 10^5$ | $2.8 \times 10^5$ | $2.1 \times 10^5$ |
| 27°C | 0 – 72 | $2.1 \times 10^4$ | $1.4 \times 10^4$ | $2.1 \times 10^4$ |
| 37°C | 0 – 48 | $1.4 \times 10^5$ | $2.8 \times 10^5$ | — |
| 27°C | 48 – 72 | – | – | $1.3 \times 10^6$ |
| 37°C | 0 – 24 | $1.6 \times 10^5$ | — | — |
| 27°C | 24 – 72 | – | $1.3 \times 10^5$ | $3 \times 10^4$ |

When the above incubation was carried out by adding substances such as insulin, indole, alloxan, indoleacetic acid, a barbiturate, amantadine, imidazole, carbachol, etc., the titer of HE—IF was found to be further enhanced. The results are shown in Table 11. The incubation was conducted at 37°C until 48 hours, but at 27°C thereafter.

TABLE 11

| Additive (conc.) | Addition time (hr.) | Interferon activity (unit/ml) | | |
|---|---|---|---|---|
| | | 24 hrs. | 48 hrs. | 72 hrs. |
| None | | $1.4 \times 10^5$ | $2.8 \times 10^5$ | $1.3 \times 10^6$ |
| Insulin | 0 | $1.9 \times 10^5$ | $3.8 \times 10^5$ | $2.9 \times 10^6$ |
| (1 U/ml) | 24 | – | $4.3 \times 10^5$ | $2.8 \times 10^6$ |
| Indole | 0 | $4 \times 10^5$ | $7.5 \times 10^6$ | $9 \times 10^5$ |
| ($10^{-2}$mM) | 24 | – | $3.1 \times 10^6$ | $6.3 \times 10^5$ |

## Example 3

A fresh amnion was cut into pieces of suitable sizes, which were then treated with 0.25% trypsin solution in a conventional manner to disperse it into single cells.

The cells were collected by centrifugation at 1,000 rpm for 10 minutes and suspended in culture mediums comprising commercially available Eagle's MEM medium (containing no serum) to which 20 $\mu$g/ml of caffeine had been added and no such additive had been added, respectively, whereby the cell numbers were adjusted to $4 \times 10^7$/ml.

To the resultant cell suspensions were added equal amounts of NDV suspensions (160 to 320 HA unit/ml). Each mixture was then subjected to shaking at slow speed at 37°C for 60 minutes thereby to cause NDV to be adsorbed onto the amnion cells.

Then, each test medium was adjusted to contain $5 \times 16^6$ cells/ml, and cultivation was conducted with slow shaking at 37°C for 24 hours. The supernatant was separated by subjecting the culture medium to centrifugation, inactivated in the same manner as in Example 1, and stored under freezing. This product was used as a HE—IF sample for assay of interferon activity, which was conducted similarly as described above. As a result, the activity was found to be $6 \times 10^4$ units/ml for tests without additive and $4.5 \times 10^5$ units/ml for tests with additive.

## Example 4

An amnion cell suspension was prepared according to the method specified in Example 2. The single cells of amnion were collected by centrifugation at 1,000 rpm for 10 minutes. The cell suspension was apportioned at $3 \times 10^6$ cells/ml in a culture medium which was a commercially available M 199 medium (pH 7.0) to which 20% calf serum had been added. While the container was rubber-stoppered, cultivation was carried out at 37°C for 5 days to form monolayers. After the medium was removed by aspiration, the cultures were inoculated with 5 ml of a NDV suspension (160 to 320 HA units/ml), and were left to stand at 37°C for 60 minutes to cause NDV to be adsorbed onto the amnion cells in the culture. After removal of the NDV suspension, 70 ml of a commercially available M 199 medium (pH 7.2) containing no serum and the same medium containing 10% calf serum were respectively added to separate cultures, and cultivation was carried out at 37°C for 24 hours. The culture media were collected, and each was centrifuged under cooling at 1,000 rpm for 10 minutes to remove cells. This step was followed by inactivation of NDV conducted similarly as in Example 1. Assaying of interferon activity by the method described above resulted in $7.5 \times 10^3$ units/ml for the serum-free medium and $1.0 \times 10^5$ units/ml for the medium containing 10% calf serum.

## Example 5

The culture medium (100 ml) prepared according to the method set forth in Example 1 was adsorbed on a column packed with 1 ml of SP-Sepharose (registered Trade Mark; Pharmacia, Inc.) and, after washing with water, eluted with an eluate containing 0.01 M $NaHPO_4$ and 0.135 M NaCl (pH 8.3). The active fractions were collected, and the interferon activity of the culture medium was found to be $10^4$ units/ml, as measured by a method of 50% reduction of VSV plaque formation wherein FL cells were used. Specific activity of the culture medium, containing 700 $\mu$g/ml of proteins, was $1.4 \times 10^4$ units/mg-protein, while that of the eluate could be raised to $2.1 \times 10^5$ units/mg-protein, that is, 15 times the former.

## Example 6

The culture medium (100 ml) used in Example 4 was adsorbed on a column packed with 1 ml of Blue Sepharose (Pharmaceia, Inc.) and eluted with a mixture containing equal amounts of an aqueous solution, containing 0.02 M $NaHPO_4$ and 1.0 M NaCl, and ethylene glycol at pH 7.2. This elution was followed by collection of the active fractions. According to this operation, specific activity was raised to about 70 times the value before the operation, that is, to $1.0 \times 10^6$ units/mg-protein, as measured by the same method as in Example 4.

## Industrial applicability

As will be apparent from the foregoing, the interferon according to the present invention derived from human amnion is highly stable and, moreover, has a high activity for inhibition of viral yield and therefore is expected to have high possibility of utilization especially for such diseases as viral infectious diseases and malignant tumors the sources of which can be found in epithelial cells.

Particularly, a typical example of human epithelial cells, namely, amnion, is available with relative ease and can be used to produce an interferon with high titer.

## Claims

1. A novel interferon, which is produced in human epithelial cells and is distinguished from human leukocyte interferon and human fibroblast interferon in reactivity with anti-HL—IF antibodies or anti-HF—IF-antibodies or both.

2. An interferon according to Claim 1, which is produced in human amnion cells, human amniotic fluid epithelial cells or human kidney epithelial cells.

3. A process for producing an interferon as claimed in Claim 1, which comprises bringing human epithelial cells into contact with an interferon inducer and then incubating said epithelial cells in a culture medium in the presence or the absence of serum.

4. A process for producing an interferon according to Claim 3, wherein the human epithelial cells is human amnion cell, human amniotic fluid epithelial cells or human kidney epithelial cells.

5. A process for producing an interferon according to Claim 3, wherein the interferon inducer is a virus.

6. A process for producing an interferon according to Claim 3, wherein there is added into the medium a pituitary hormone, insulin, a xanthine derivative, alloxan, indole, indoleacetic acid, a barbiturate, amantadine, imidazole or carbachol.

7. A process for producing an interferon according to any of Claims 3 to 6, wherein the incubation is carried out at 37°C for 38 to 58 hours and thereafter at $27 \pm 2$°C.

## Revendications

1. Nouveau interféron, qui est produit dans des cellules épithéliales et se distingue de l'interféron leucocytaire humain et de l'interféron fibroblaste en réactivité avec les anticorps anti-HL—IF ou les anticorps anti-HF—IF ou les deux.

2. Interféron selon la revendication 1, qui est produit dans des cellules de l'amnios humain ou

# 0 048 283

dans des cellules épithéliales du fluide amniotique humain ou des cellules épithéliales du rein humain.

3. Procédé de production d'un interféron selon la revendication 1, qui consiste à mettre des cellules épithéliales humaines en contact avec un inducteur d'interféron et à incuber ensuite lesdites cellules épithéliales dans un milieu de culture en présence ou en l'absence de sérum.

4. Procédé de production d'un interféron selon la revendication 3, dans lequel les cellules épithéliales humaines sont des cellules de l'amnios humain, des cellules épithéliales du fluide amniotique humain ou des cellules épihthéliales du rein humain.

5. Procédé de production d'un interféron selon la revendications 3, dans lequal l'inducteur d'interféron est un virus.

6. Procédé de production d'un interféron selon la revendication 3, dans lequel on ajoute au milieu une hormone pituitaire, de l'insuline, une dérivée de xanthine, de l'alloxane, de l'indole, de l'acide indoleacétique, une barbiturate, de l'amantadine, de l'imidazole, ou du carbachole.

7. Procédé de production d'un interféron selon l'une quelconque des revendications 3 à 6, dans lequel l'incubation s'effectue à 37°C pendant 38 à 58 heures et ensuite à 27 ± 2°C.

**Patentansprüche**

1. Neues Interferon, das in menschlichen Epithelzellen produziert wird und sich von menschlichem Leukozyten-Interferon und menschlichem Fibroblast-Interferon bezüglich der Reativität mit Anti-HL—IF-Antikörpern oder Anti-HF—IF-Antikörpern oder beidem unterscheidet.

2. Interferon nach Anspruch 1, das in menschlichen Amnionzellen, menschlichen Fruchtwasser-Epithelzellen oder menschlichen Nieren-Epithelzellen produziert wird.

3. Verfahren zur Herstellung eines Interferons nach Anspruch 1, das das Inkontaktbringen menschlicher Epithelzellen mit einem Interferonauslöser und darauffolgendes Inkubieren der Epithelzellen in einem Kulturmedium in Anwesenheit oder Abwesenheit von Serum einschließt.

4. Verfahren zur Herstellung eines Interferons nach Anspruch 3, bei dem die menschlichen Epithelzellen menschliche Amnionzellen, menschliche Fruchtwasser-Epithelzellen oder menschliche Nieren-Epithelzellen sind.

5. Verfahren zur Herstellung eines Interferons nach Anspruch 3, bei dem der Interferonauslöser ein Virus ist.

6. Verfahren zur Herstellung eines Interferons nach Anspruch 3, bei dem in das Medium ein Hypophysenhormon, Insulin, ein Xanthinabkömmling, Alloxan, Indol, Indolyl-3-Essigsäure, ein Barbiturat, Amantadin, Imidazol oder Carbachol zugefügt wird.

7. Verfahren zur Herstellung eines Interferons nach einem der Ansprüche 3 bis 6, bei dem die Inkubation bei 37°C für 38 bis 58 Stunden und danach bei 27 ± 2°C ausgeführt wird.

13

F I G. I

# FIG. 2